Europäisches Patentamt

**European Patent Office**

**Office européen des brevets**

(11) Publication number: **0 193 681**
A2

(12) **EUROPEAN PATENT APPLICATION**

(21) Application number: **85309341.7**

(22) Date of filing: **20.12.85**

(51) Int. Cl.⁴: **A 61 F 2/30**

(30) Priority: **20.12.84 GB 8432266**

(43) Date of publication of application: **10.09.86**
**Bulletin 86/37**

(84) Designated Contracting States: **BE CH DE FR GB IT LI NL SE**

(71) Applicant: **Chas F Thackray Limited, P.O. Box HP 171 1 Shire Oak Street, Leeds LS6 2DP West Yorkshire (GB)**

(72) Inventor: **Elloy, Martin Arthur, Beechwood House Church Street, Church Fenton North Yorkshire (GB)**

(74) Representative: **Harrison, Michael Robert et al, Urquhart-Dykes & Lord 5th Floor Tower House Merrion Way, Leeds, LS2 8PA (GB)**

(54) **Joint prostheses.**

㊿ A joint prothesis comprises a first component (11), a second component (17) and a plastics sleeve (13) lying between the first and second components. The second component (17), together with the sleeve (13) is an interference fit within a blind bore in the first component (11), to key the first and second components together.

EP 0 193 681 A2

## JOINT PROSTHESES

This invention is concerned with joint prostheses, for instance prostheses which may be used in connection with artificial hip, knee, shoulder and other joints. In such artificial joints it is sometimes desirable to use parts made of different substances, the mechanical properties of which are such that it is necessary to prevent them having direct contact with each other. Examples of such substances are ceramic materials which may provide good bearing surfaces and metals which provide good anchoring portions of the prosthesis.

In order to keep separate those components of a prosthesis being made of imcompatible materials, it has been proposed to provide an interposing plastics component. UK Patent Specification No 1593819 describes an arrangement in which the three components, the ceramic head, the metal stem and the interposing plastic member, are held together by means of a secondary device, in this case a screw. As a result the plastics member behaves only as a compliant load distributing, dimensional change accommodating medium.

According to the present invention there is provided a prosthesis for replacing part of a natural joint, said prosthesis comprising a first component having a bearing surface for mating with a corresponding bearing surface of another part of the joint, a second component having at one end an elongated portion for fixing within a natural bone and having at the opposite end a substantially cylindrical head for location within a substantially cylindrical blind bore in the first component, and a third component comprising a plastics sleeve located within the bore to separate said first and second portions, the prosthesis being arranged so that the three portions are held together, and relative rotation therebetween prevented, solely by the deformation of the third component between said first and second components, said deformation acting to key the third component to the first and second components.

The plastics sleeve is preferably very thin, and is preferably less than 1,000 microns thick.

Surprisingly, it has been found that a very small difference between the external diameter of the cylindrical head, including the plastics sleeve, and the internal diameter of the blind bore is required to cause the first and second components to key together.

Preferably, the external diameter of the cylindrical head of the second component including the thickness of the plastics sleeve is greater than the internal diameter of the blind bore in the first component to provide an interference fit, the difference between the diameters being between 50 and 250 microns.

Typically the first component may be made of a ceramics material and the second component made of a metal or metal alloy. The interposing plastics sleeve minimises the stresses imposed on the weaker ceramic component. The first and second components are electrically insulated from each other by means of the plastics sleeve thereby minimising the risk of electrolytic corrosion which might otherwise occur where dissimilar metals are used for both components. The plastics material is preferably an ultra high molecular weight polyethylene or a cross-linked high density polyethylene.

As a result of the use of a plastics sleeve there is a reduction in dimensional accuracy required for satisfactory fixation of the first and second components to each other and thereby a reduction in the cost of production of the prosthesis. The use of the plastics sleeve allows for accommodation of the different mechanical responses of materials with unmatched mechanical properties, when the prosthesis is subjected to loading or temperature changes.

In accordance with the present invention, the plastics material is designed to deform to key into surface roughness or other regular or irregular non-smooth features of the first and second components in order to prevent relative movement at each material interface. The deformation may occur during the assembly of the prosthesis because of the close interference fit of the components and/or from physiological loading of the prosthesis in use.

The blind bore is essentially cylindrical, tapered surfaces being avoided so that bursting forces are not produced in the female (first) component which would otherwise adversely affect its fatigue life.

The blind bore is preferably made only just deep enough to give an appropriate relationship with the line(s) of action of the applied load so as give the maximum continuous section of material in the weaker female component in order to withstand internal bursting forces generated during loading.

Preferably the third portion does not occupy the whole of the bore between the first and second components. The plastic sleeve is preferably arranged so that the hydrostatic pressure generated during loading is transmitted only to the deeper part of the internal surface of the female (first) component so that the stresses induced are thereby reduced. This may be achieved by utilising a plastics sleeve which terminates some distance short of the entrance to the bore. Alternatively the plastics sleeve may be provided with recesses or cut out portions. In a further alternative, recesses may be formed in the first or second components, such recesses opening into the bore. Such recesses, whether provided in either or both the female or male surfaces, should be located at the desired extremity of pressurisation to accommodate plastic flow during loading and thus prevent the transmission of a hydrostatic pressure beyond that point.

4

0193681

These pressure relieving cavities may be filled with air, another gas or some other suitably compliant medium.

Embodiments of the present invention will now be described, by way of examples only, and with reference to the accompanying drawings, in which:-

Figure 1 illustrates one embodiment of the present invention;

Figure 2 illustrates another embodiment of the present invention; and,

Figure 3 illustrates further embodiments of the plastics sleeve.

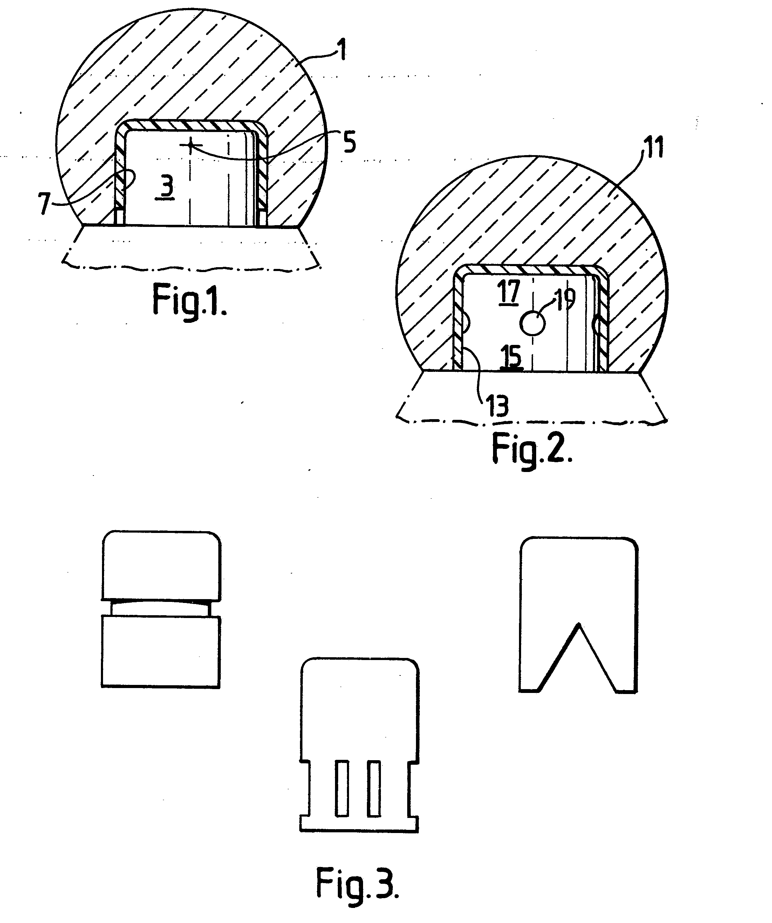

Referring to Figure 1, the ceramic head 1 of a femoral prosthesis has an essentially flat bottomed central bore 3 extending just byond the centre point 5 of the sphere. The stem (not shown) of the prosthesis has a corresponding parallel male portion of slightly smaller cross sectional dimension which is provided with a thin walled plastics sleeve 7, the combination being a push fit within the bore of the head 1. The relative thinness of the sleeve 7 resits extrusion under the influence of the hydrostatic pressure generated by the load.

Sleeve 7 is made sufficiently short so that it does not reach the mouth of the bore. In this way the hydrostatic pressure generated is only applied to the deeper region of the bore thereby limiting bursting forces while retaining the strength of the full length head component.

The diameter of the ceramic heas 1 is 22.25 mm and the distance of the centre of the head 1 to the mouth of the bore is 8.5 mm. The depth of the bore is 11.5 mm and the diameter is 11 mm. The sleeve 7 is shortened such that its edges are 3.5 mm from the mouth of the bore. The thickness of the sleeve is 1/4 mm.

Referring to Figure 2, an alternative embodiment of a femoral prosthesis in accordance with the present invention includes a ceramic head 11, a metal stem 15 and an intermediate plastics sleeve 13. Spigot 17 of stem 15 extends into the blind bore of head 11 with the plastics sleeve interposed between the spigot and head. Recesses 19 are formed in spigot 17 at a level lying well within the bore of the head and typically at about the halfway point. The sleeve is such that it does not fill these recesses so that cavities are left after assembly of the prosthesis from the three components. These recesses or cavities allow for extrusion of the plastics material into them during loading and thereby prevent the transmission of hydrostatic pressure to the bore from the position of the recesses to the open end of the bore. In this way bursting forces are limited without loss of strength of the ceramic components while at the same time there is maintained a neat and possibly hermetically sealed assembly.

Figure 3 shows alternative embodiments of the plastics sleeve. The sleeve may include different shaped recesses or just a lateral groove which gives a break in the surface.

In any of the embodiments, the spigot 17 plus the sleeve 13 is an interference fit within the bore 3, and it has surprisingly been found that an external diameter of spigot 17 plus sleeve 13 which is only 50 microns larger than the internal diameter of the bore 3 is sufficient to key the spigot 17 to the bore 3. A different of up to 250 microns could be tolerated.

In the above-described embodiments the components can be fitted together without grinding out of the bore of the ceramic head after firing of the latter. Accordingly, the ceramic head is not subjected to the risk of microcracking during the grinding processes.

By virtue of the present invention, frozen stresses are eliminated from the weaker component, such as the ceramic head of the above-described femoral prostheses.

Bursting pressures are greatly reduced in prostheses of the present invention.  As a result, femoral prostheses with ceramic heads having sizes as low as 22 mm diameter may be produced, such prostheses having impact strengths not reduced below that currently achieved with 32 mm heads attached by means of a taper lock technique.  Use of such a technique requires a high degree of dimensional accuracy in both the head and the stem components which add significantly to their cost of production, particularly in the case of ceramic heads.  The necessary grinding of the ceramic components may, as indicated above, also lead to microcracking and thereby a further reduction in their strength.

Other material combinations such as titanium and stainless steel may be used wihtout the risk of setting up an electrolytic cell with its consequent risk of corrosion.

1

CLAIMS

1. A prosthesis for replacing part of a natural joint, said prosthesis comprising a first component (1) having a bearing surface for mating with a corresponding bearing surface of another part of the joint, a second component having at one end an elongated portion for fixing within a natural bone and having at the opposite end a substantially cylindrical (17) head for location within a substantially cylindrical blind bore (3) in the first component, and a third component comprising a plastics sleeve located within the bore to separate said first and second portions, characterised in that the prosthesis is arranged so that the three portions are held together, and relative rotation therebetween prevented, solely by deformation of the third component between said first and second components, said deformation acting to key the third component to the first and second components.

2. A prosthesis according to Claim 1 characterised in that the plastics sleeve (13) is less than 1000 microns thick.

3. A prosthesis according to Claim 1 or Claim 2 characterised in that the external diameter of the cylindrical head of the second component including the thickness of the plastics sleeve (13) is greater than the internal diameter of the blind bore (3) in the first component to provide an interference fit, the difference between the diameters being between 50 and 250 microns.

4. A prosthesis according to any of the preceding claims characterised in that the blind bore in the first component (1) is made only just deep enough to give an appropriate relationship with the line or lines of action of the applied load so as to give the maximum continuous section of material in the first component.

5. A prosthesis according to any of the preceding claims, characterised in that the third component (13) does not occupy the whole of the bore (3) in the first component (1).

6. A prosthesis according to Claim 5 characterised in that it is arranged so that the hydrostatic pressure generated during loading thereof is transmitted only to the deeper part of the internal surface of the first component (1).

7. A prosthesis according to Claim 5 or Claim 6 characterised in that the plastics sleeve (13) terminates some distance short of the entrance to the bore (3).

8. A prosthesis according to Claim 5 or Claim 6 characterised in that the plastics sleeve is provided with recesses.

9. A prosthesis according to Claim 5 or Claim 6 characterised in that either or both of the first and second components have recesses (19) formed therein, said recesses opening out into the bore (3).

10. A prosthesis according to any of the preceding claims characterised in that the plastics sleeve (13) is made of ultra high molecular weight polyethelene.

11. A prosthesis according to any of the preceding claims characterised in that the plastics sleeve (13) is made of a cross-linked high density polyethelene.

Fig.1.

Fig.2.

Fig.3.